(19) Europäisches Patentamt / European Patent Office / Office européen des brevets

(11) **EP 1 925 278 A1**

(12) **DEMANDE DE BREVET EUROPEEN**

(43) Date de publication:
**28.05.2008 Bulletin 2008/22**

(21) Numéro de dépôt: **07120902.7**

(22) Date de dépôt: **16.11.2007**

(51) Int Cl.:
*A61K 8/02* (2006.01)       *A61K 8/19* (2006.01)
*A61K 8/25* (2006.01)       *A61K 8/29* (2006.01)
*A61Q 1/02* (2006.01)       *A61Q 1/04* (2006.01)
*A61Q 1/06* (2006.01)       *A61Q 1/10* (2006.01)
*A61Q 3/02* (2006.01)

(84) Etats contractants désignés:
**AT BE BG CH CY CZ DE DK EE ES FI FR GB GR HU IE IS IT LI LT LU LV MC MT NL PL PT RO SE SI SK TR**
Etats d'extension désignés:
**AL BA HR MK RS**

(30) Priorité: **17.11.2006 FR 0654976**
**17.11.2006 FR 0654977**
**17.11.2006 FR 0654981**
**12.12.2006 FR 0655453**
**12.12.2006 FR 0655454**
**12.12.2006 FR 0655460**

(71) Demandeur: **L'ORÉAL**
**75008 Paris (FR)**

(72) Inventeur: **Thevenet, Ludovic**
**92340, BOURG LA REINE (FR)**

(74) Mandataire: **Tanty, François**
**Nony & Associés,**
**3, rue de Penthièvre**
**75008 Paris (FR)**

(54) **Gamme de compositions cosmétiques**

(57)    La présente invention concerne une gamme de compositions cosmétiques comportant au moins deux compositions comportant chacune un milieu cosmétiquement acceptable et au moins un pigment interférentiel multicouche, chaque composition étant de couvrance supérieure ou égale à 25,

les pigments interférentiels multicouche respectifs des différentes compositions étant choisis de manière à ce que les couleurs des compositions dans la masse, prises deux à deux, diffèrent d'un écart $\Delta E_{avant\ application}$ inférieur ou égal à 3 et que les compositions après application présentent, prises deux à deux, un écart de couleur $\Delta E_{après\ application}$ supérieur ou égal à 5.

## Description

La présente invention concerne les compositions cosmétiques, et plus particulièrement celles destinées au maquillage des matières kératiniques.

## Arrière-plan

Il est connu d'introduire dans les compositions de maquillage des pigments diffusants pour produire des couleurs par un phénomène d'absorption de la lumière par des chromophores spécifiques.

A ces pigments diffusants, qui sont nécessaires pour bénéficier d'un fond coloré continu et suffisamment couvrant, peuvent être ajoutées des particules à effet(s) pour créer par exemple des points de brillance ou conférer un aspect nacré.

Toutefois, l'intensité de la couleur produite par ces compositions peut s'avérer insuffisante pour conduire à un résultat complètement satisfaisant.

Les pigments interférentiels multicouche, comportant un empilement de couches dont les indices de réfraction et les épaisseurs sont convenablement choisis afin de générer une couleur par un phénomène d'interférences, permettent de produire une intensité de couleur plus importante que les pigments diffusants ci-dessus.

A la connaissance de l'inventeur, dans les compositions disponibles commercialement, à l'exception des poudres, ces pigments interférentiels multicouche sont employés à une concentration massique n'excédant pas 5 %.

Il est connu par ailleurs par les fards à paupières de marque CHRYSALIDE de la société LANCÔME de conférer à la fois couvrance et intensité colorielle au moyen d'un maquillage faisant intervenir un premier geste consistant à déposer sur les matières kératiniques une couche de base de couleur noire contenant un pigment diffusant apportant de la couvrance et de déposer ensuite par un second geste sur cette couche de base une composition apportant de la couleur par le biais d'un pigment interférentiel multicouche. Sans la couche de base, la couche de recouvrement est pratiquement invisible car non couvrante et sans couleur.

Le recours à deux applications successives complique le maquillage et rend le conditionnement plus coûteux.

## Résumé

Gammes de compositions

Il existe un besoin pour bénéficier de compositions capables d'apporter de la couvrance et de produire une couleur saturée, afin de pouvoir obtenir en un seul geste un maquillage à la fois couvrant et coloré.

Par ailleurs, il est souhaitable de bénéficier de compositions cosmétiques destinées au maquillage des matières kératiniques qui présentent de nouveaux effets susceptibles d'attirer les consommateurs, sans que ces nouveaux effets ne se produisent au détriment de la qualité du maquillage obtenu.

L'invention vise notamment à répondre à tout ou partie des besoins identifiés ci-dessus.

La présente invention a pour objet, selon l'un de ses aspects, une gamme de compositions cosmétiques comportant au moins deux compositions comportant chacune un milieu cosmétiquement acceptable et au moins un pigment interférentiel multicouche, les compositions étant chacune de couvrance supérieure ou égale à 25, mieux 30, les pigments interférentiels multicouche respectifs des différentes compositions étant choisis de manière à ce que les couleurs des compositions dans la masse, prises deux à deux, diffèrent d'un écart $\Delta E_{avant\ application}$ inférieur ou égal à 3 et que les compositions après application présentent, prises deux à deux, un écart de couleur $\Delta E_{après\ application}$ supérieur ou égal à 5.

La couleur après application est déterminée pour les besoins du calcul de $\Delta E$ après étalement de la composition sur une carte de contraste, comme pour la mesure de la couvrance.

Par « gamme », il faut comprendre une pluralité de compositions proposée aux consommateurs, par exemple sous la même marque et dans un conditionnement.

La gamme selon l'invention peut être complétée dans l'offre aux consommateurs par une gamme plus large comprenant des compositions ne correspondant pas à l'invention, proposées par exemple sous la même marque et dans le même conditionnement que les compositions selon l'invention.

Les compositions de la gamme peuvent être toutes anhydres ou toutes aqueuses.

$\Delta E_{avant\ application}$ peut être inférieur ou égal à 2,

$\Delta E_{après\ application}$ peut être supérieur ou égal à 5, mieux 6.

La couvrance peut être supérieure à 25, par exemple comprise entre 30 et 70, par exemple supérieure à toute valeur entière comprise dans cet intervalle, par exemple supérieure ou égale à 40.

Chaque composition peut présenter un écart de couleur entre la couleur dans la masse et la couleur après application supérieur ou égal à 5, par exemple supérieur à l'une des valeurs entières comprises dans l'intervalle 5 à 30.

Avec les pigments interférentiels multicouche, la production de la couleur par le phénomène d'interférences est en compétition avec la production de la couleur par le phénomène d'absorption par la couche de surface du pigment.

Ainsi, lorsque la concentration en pigment augmente suffisamment, la couleur produite par le phénomène d'interférences diminue au profit de celle produite par absorption.

L'invention, en exploitant cette propriété, permet d'observer une variation de couleur de chaque composition de la gamme à l'application, qui confère un aspect ludique à l'utilisation. Ce caractère ludique est renforcé en ce que toutes les compositions de la gamme présentent sensiblement la même couleur dans la masse.

L'invention offre également de nouvelles possibilités en matière de commercialisation des compositions cosmétiques, en permettant de jouer au niveau du conditionnement sur cette variation de couleur avant et après application.

La couleur de chaque composition de la gamme, dans la masse, peut être blanche. Par « blanc » il faut comprendre achromatique au sens de la CIE.

Chaque composition de la gamme, en masse, peut avoir un indice de blancheur supérieur ou égal à 40.

Chaque composition de la gamme peut ne pas comporter d'autre agent de coloration que le ou les pigments interférentiels multicouche.

Au moins un pigment interférentiel multicouche des compositions de la gamme peut comporter au moins quatre couches, par exemple.

Une composition de la gamme peut comporter deux pigments interférentiels multicouche ayant des couches constituées des mêmes matériaux, au moins une couche d'un pigment ayant une épaisseur différente d'une couche correspondante de l'autre pigment, de façon à produire des couleurs différentes.

Les compositions de la gamme peuvent comporter chacune au moins un pigment interférentiel multicouche ayant un substrat comportant un oxyde de fer ou d'aluminium.

Les pigments interférentiels multicouche de toutes les compositions de la gamme peuvent avoir les mêmes dimensions, à 20 % près.

Les pigments interférentiels multicouche de toutes les compositions de la gamme peuvent avoir un substrat de même nature et/ou une couche de surface de même nature. Cela peut faciliter l'obtention d'une même couleur dans la masse.

Deux compositions au moins peuvent ne différer entre elles que par l'épaisseur d'au moins l'une des couches interférentielles de leurs pigments interférentiels respectifs.

Le nombre de compositions de la gamme peut être compris entre 2 et 8, par exemple.

Toutes les compositions de la gamme peuvent être conditionnées dans des récipients identiques.

Chaque composition peut être conditionnée avec des moyens indiquant la couleur de la composition après l'application sur des matières kératiniques (lèvres, peau, ongles, cils, sourcils, cheveux).

Il peut s'agir par exemple d'une impression d'une encre ou d'un vernis, d'une couche mince de composition ou d'un moulage ou surmoulage d'un matériau incorporant le ou les pigments interférentiels multicouche.

L'invention a encore pour objet, selon l'un de ses aspects, une gamme de compositions cosmétiques comportant au moins deux compositions comportant chacune un milieu cosmétiquement acceptable et au moins un pigment interférentiel multicouche en une teneur massique supérieure ou égale à 7 %, les pigments interférentiels générant des couleurs différentes à l'application, les pigments interférentiels étant de préférence à substrat non opaque, notamment de verre, silice, mica, et comportant de préférence aucune couche colorée, la teneur en pigment diffusant générant une couleur par absorption dans chaque composition étant de préférence inférieure à 0,5 %.


Compositions blanches dans la masse


De telles compositions peuvent être utilisées pour former une gamme de compositions selon l'invention. De telles compositions peuvent également avoir un intérêt isolément.

L'invention a encore pour objet, selon l'un de ses aspects, indépendamment ou en combinaison avec ce qui précède, une composition cosmétique solide, notamment en stick, comportant, dans un milieu cosmétiquement acceptable, au moins un pigment interférentiel multicouche apte à conférer à la composition une couleur blanche dans la masse, et à provoquer après application une variation de couleur ΔE de la composition d'au moins 5.

Par « stick », encore appelé parfois « raisin » ou « bâton », on désigne une composition sous forme solide, généralement sous forme de bloc allongé, permettant un transfert sur les matières à maquiller par friction. Un stick peut être obtenu par exemple par moulage ou extrusion.

La couleur après application est déterminée pour les besoins du calcul de ΔE après étalement de la composition sur une carte de contraste, comme pour la mesure de la couvrance.

La composition, en masse, peut avoir un indice de blancheur supérieur ou égal à 40.

La composition peut être anhydre ou aqueuse.

Selon les compositions, l'écart ΔE peut être par exemple compris entre 5 et 30, notamment être supérieur à toute valeur entière comprise dans cet intervalle.

EP 1 925 278 A1

La couvrance de la composition peut être comprise entre 30 et 70, notamment être supérieure à toute valeur entière comprise dans cet intervalle, par exemple supérieure ou égale à 40.

La teneur massique en pigment interférentiel multicouche peut aller de 7 à 20 %, mieux 8 à 15 %, notamment dans le cas d'une composition non pulvérulente, par exemple liquide ou en stick.

Pour une composition pulvérulente, libre ou compactée, la teneur en pigment interférentiel multicouche va par exemple de 40 à 95 %, mieux 50 à 80 %.

La composition peut ne pas comporter d'autre agent de coloration que le ou les pigments interférentiels multicouche.

Le pigment interférentiel multicouche peut comporter au moins quatre couches, par exemple.

Le pigment interférentiel multicouche peut comporter un substrat en une matière plus ou moins rugueuse, ce qui peut permettre de conférer plus ou moins de brillance à la composition.

Le substrat est par exemple choisi en mica naturel, relativement rugueux, en mica synthétique, en alumine, silice ou en verre ou métal pour une surface plus lisse.

La composition peut comporter deux pigments interférentiels multicouche ayant des couches constituées des mêmes matériaux, au moins une couche d'un pigment ayant une épaisseur différente d'une couche correspondante de l'autre pigment, de façon à produire des couleurs différentes.

L'invention a encore pour objet, selon un autre de ses aspects, indépendamment ou en combinaison avec ce qui précède, une composition cosmétique comportant, dans un milieu cosmétiquement acceptable, au moins un pigment interférentiel multicouche apte à conférer à la composition une couleur blanche dans la masse, d'indice de blancheur supérieur ou égal à 40, la teneur massique total en pigment interférentiel multicouche allant de 7 à 20 % dans le cas d'une composition non pulvérulente et de 40 à 95 %, mieux 40 à 80%, dans le cas d'une composition pulvérulente.

L'invention a encore pour objet, selon un autre de ses aspects, indépendamment ou en combinaison avec ce qui précède, un ensemble de conditionnement comportant :

- une composition blanche dans la masse telle que définie ci-dessus,
- un moyen permettant de renseigner l'utilisateur sur la couleur de la composition après application sur des matières kératiniques (peau, lèvres, cheveux, ongles cils, sourcils). Il peut s'agir par exemple d'une impression d'une encre ou d'un vernis, d'une couche mince de composition ou d'un moulage ou surmoulage d'un matériau incorporant le ou les pigments interférentiels multicouche.

Ensemble pour application bi-couche

L'invention a encore pour objet, selon l'un de ses aspects, indépendamment ou en combinaison avec ce qui précède, un ensemble comportant :

- une première composition cosmétique à appliquer sur des matières kératiniques, cette première composition étant par exemple l'une des compositions de la gamme ci-dessus, comportant dans un milieu cosmétiquement acceptable au moins un pigment interférentiel multicouche, la première composition présentant une couvrance supérieure ou égale à 25, mieux 30, la teneur en pigment interférentiel multicouche conférant à la composition un changement de couleur $\Delta E$ d'au moins 2, mieux 5 entre la couleur dans la masse et celle après application sur les matières kératiniques,
- une deuxième composition appelée composition de recouvrement ou *top coat,* à appliquer sur la première, ou
- une deuxième composition appelée composition de base ou *base coat,* à appliquer avant la première sur les matières kératiniques.

La deuxième composition est de préférence liquide.

La deuxième composition peut comporter une phase grasse, afin de conférer de la brillance au maquillage.

La deuxième composition peut être transparente, afin de ne pas affecter la saturation de la couleur produite par la première composition.

La deuxième composition peut être dépourvue de corps solides, afin de ne pas diffuser la lumière réfléchie par la première composition. En variante, la deuxième composition peut comporter au moins un pigment à effet, notamment un pigment réfléchissant métallique, des pigments interférentiels, des composés à propriétés thermochromiques, photochromiques, tribochromiques, piézochromes, solvatochromes ou mécano luminescentes.

La première composition peut être anhydre ou aqueuse.

Selon les premières compositions, l'écart $\Delta E$ peut être supérieur à toute valeur entière comprise dans l'intervalle 5 à 30.

La couvrance de la première composition ou de la composition de base peut être supérieure à 30, notamment être supérieure à toute valeur entière comprise dans l'intervalle 30 à 70, par exemple supérieure ou égale à 40.

La teneur massique en pigment interférentiel multicouche dans la première composition peut aller de 7 à 15 %, mieux 8 à 15 %, notamment dans le cas d'une première composition non pulvérulente, par exemple liquide ou en stick.

Pour une première composition pulvérulente, la teneur en pigment interférentiel multicouche va par exemple de 40 à 95 %, mieux 40 à 80 %.

La couleur de la première composition dans la masse peut être blanche. La composition de base, en masse, peut avoir un indice de blancheur supérieur ou égal à 40.

La première composition peut ne pas comporter d'autre agent de coloration que le ou les pigments interférentiels multicouche.

La première composition peut comporter deux pigments interférentiels multicouche ayant des couches constituées des mêmes matériaux, au moins une couche d'un pigment ayant une épaisseur différente d'une couche correspondante de l'autre pigment, de façon à produire des couleurs différentes.

L'ensemble peut comporter un moyen pour indiquer la couleur après application de la première composition ou après application des deux compositions de l'ensemble, sur les matières kératiniques (peau, lèvres, ongles, cils, sourcils, cheveux).

Il peut s'agir par exemple d'une impression d'une encre ou d'un vernis, d'une couche mince de composition ou d'un moulage ou surmoulage d'un matériau incorporant le ou les pigments interférentiels multicouche.

La deuxième composition de recouvrement comporte un milieu cosmétiquement acceptable.

La formulation de ce milieu est choisie de façon à permettre l'application sur la première composition, afin par exemple de conférer de la brillance et/ou améliorer la tenue et/ou nuancer un effet optique apporté par la première composition.

La deuxième composition de recouvrement peut comporter un corps gras liquide ou un agent filmogène.

La deuxième composition de recouvrement peut comporter un colorant et un ou plusieurs actifs et autres composés.

La deuxième composition de recouvrement peut présenter toute forme galénique compatible avec l'application sur la première composition.

Lorsque l'obtention de brillance est recherchée, la composition de recouvrement est de préférence liquide et transparente, comportant avantageusement une phase grasse.

La deuxième composition peut comporter au moins un pigment à effet. Ce pigment sera dans une teneur qui n'affecte pas l'observation de la couleur produite par la composition de base une fois appliquée sur les matières kératiniques.

Chaque composition peut être conditionnée dans tout réceptacle ou sur tout support prévu à cet effet. Les première et deuxième compositions peuvent être contenues, le cas échéant, dans deux compartiments d'un même dispositif de conditionnement et/ou dans un même emballage avant la première utilisation.

Chaque composition peut être appliquée en utilisant un applicateur, floqué ou non, par exemple une mousse, un embout, un pinceau, un feutre, une spatule, un fritté, une brosse, un peigne, un tissé ou non tissé.

L'application peut encore s'effectuer avec le doigt ou en déposant directement chaque composition sur le support à maquiller, par exemple par friction dans le cas d'un stick ou par pulvérisation ou projection à l'aide d'un dispositif piézoélectrique ou électrostatique ou par transfert d'une couche de composition préalablement déposée sur un support intermédiaire.

La première composition peut être conditionnée dans un dispositif de conditionnement qui laisse apparaître la couleur de la composition dans la masse.

Il peut s'agir d'un récipient ayant un corps au moins partiellement transparent et/ou d'un dispositif de conditionnement comportant un organe de bouchage au moins partiellement transparent.

La première composition peut être conditionnée dans un dispositif qui permet de voir à la fois la couleur de la composition dans la masse et la couleur de la composition après application sur des matières kératiniques.

La première composition peut encore être conditionnée dans un dispositif qui comporte des moyens représentatifs de la couleur révélée à l'application, par exemple un dépôt d'une couche de la première composition ou d'une encre ou vernis comportant les mêmes agents de coloration que la première composition.

Le cas échéant, la première composition ou la deuxième composition de recouvrement peut être conditionnée avec un aimant permettant de modifier l'orientation des particules de pigment interférentiel multicouche, lorsque ce pigment présente une susceptibilité magnétique non nulle.

**Mesure de la couvrance**

Compositions liquides (à 25°C)

Par « composition liquide », on entend une composition dont on peut mesurer la viscosité. Une composition liquide peut s'écouler sous l'effet de son propre poids.

La couvrance des compositions est mesurée à épaisseur finie de 50 μm, les compositions liquides étant par exemple des compositions à appliquer sur les lèvres, notamment des rouges à lèvres liquides, brillants à lèvres liquides et baumes à lèvres liquides, des vernis à ongles, des fards à paupières, des fonds de teint liquides, des mascaras et autres produits de maquillage liquides non destinés à être à appliqués sur les lèvres.

La composition est étalée sur des cartes de contraste noir mat et blanc mat, par exemple de marque LENETA Form WP1 pour la carte noir mat et Leneta 1A pour la carte blanc mat.

L'application peut s'effectuer avec un étaleur automatique.

Les mesures de couvrance s'effectuent sur les compositions ainsi étalées.

Compositions solides (à 25°C)

Les compositions solides sont celles dont on ne peut mesurer la viscosité.

Il peut s'agir de compositions coulées en stick ou pulvérulentes, sous forme de poudres libres ou compactées.

a) Pour les compositions solides pulvérulentes, libres ou compactées, la composition est appliquée en utilisant les mêmes cartes de contraste que ci-dessus, recouvertes d'un ruban adhésif transparent, légèrement rugueux, par exemple de marque BLENDERM® de la société 3M et de référence 15025, collé par la face adhésive sur les cartes de contraste.

La composition est déposée sur le ruban adhésif de manière à obtenir un dépôt homogène de 0,5 mg/cm$^2$ ± 0,02 mg/cm$^2$.

Il est possible d'utiliser pour effectuer le dépôt une éponge chargée avec la composition et montée sur un appareil de délitage qui fait effectuer des mouvements prédéfinis à l'éponge. L'éponge est par exemple une éponge à usage unique de type « LANCÔME - Photogenic », utilisée du coté rose.

b) Les compositions en stick sont fondues, par exemple à 90°C, puis étalées à l'état liquide avec une épaisseur de 50 μm sur des cartes de contraste noir mat et blanc mat, par exemple de mêmes références que ci-dessus, non recouvertes de BLENDERM®. Le barreau d'étalement est maintenu à la même température que la composition, de façon à éviter un choc thermique.

Mesures et calculs

Des spectres de réflectance sont acquis à l'aide d'un spectrocolorimètre MINOLTA 3700-d (géométrie de mesure diffuse/8° et observation D65/10°, mode composante spéculaire exclue, petite ouverture (CREISS)) sur les fonds noir et blanc, les cartes de contraste étant éventuellement recouvertes de BLENDERM® comme indiqué ci-dessus.

Les spectres sont exprimés en coordonnées colorimétriques dans l'espace CIELab76 au sens de la Commission Internationale de l'Eclairage selon la recommandation 15:2004.

Le contraste ratio, ou couvrance, est calculé en faisant la moyenne arithmétique de Y sur fond noir, divisée par la valeur moyenne de Y sur fond blanc, multiplié par 100.

**Mesure de la couleur de la composition dans la masse**

La couleur dans la masse est mesurée après avoir rempli un contenant présentant une profondeur suffisamment importante pour pouvoir considérer une épaisseur de produit infinie au vu de la mesure, par exemple une profondeur de 3 mm ou plus.

Les coordonnées L*, a* et b* sont mesurées avec un spectrocolorimètre, par exemple de marque MINOLTA CM-2002 (D65/10°, mode composante spéculaire exclue).

**Mesure de la couleur après application**

La couleur est mesurée sur le fond sombre de la carte de contraste, la composition étant étalée comme pour la mesure de la couvrance ainsi que détaillé ci-dessus mais à une épaisseur de 150 μm au lieu de 50 μm pour les compositions liquides et non pulvérulentes, notamment en stick.

L'écart ΔE est calculé comme suit :

$$\Delta E = \left[ \left( a^*_{dans\,la\,masse} - a^*_{après\,application} \right)^2 + \left( b^*_{dans\,la\,masse} - b^*_{après\,application} \right)^2 + \left( L^*_{dans\,la\,masse} - L^*_{après\,application} \right)^2 \right]^{1/2}$$

Lorsque l'écart ΔE varie en fonction de l'angle d'observation en raison de la présence d'un agent de coloration goniochromatique, c'est l'écart maximal qui est retenu.

### Mesure de l'indice de blancheur

L'indice de blancheur est calculé d'après les mesures de couleur à épaisseur infinie et selon la norme ASTM E313-05.

### Pigment interférentiel multicouche

L'expression « pigment interférentiel multicouche » désigne un pigment capable de produire une couleur par un phénomène d'interférences entre les rayonnements lumineux réfléchis par une pluralité de couches superposées d'indices de réfraction différents, notamment une succession de couches de haut et de bas indices de réfraction. Le pigment peut comporter un substrat, par exemple de mica, recouvert d'une seule couche d'indice de réfraction différent, par exemple de $TiO_2$.

Tous les pigments interférentiels multicouche peuvent être envisagés.

La couleur produite par le pigment interférentiel multicouche peut être quelconque, étant par exemple de longueur d'onde dominante comprise entre 580 et 650 nm ou non.

La composition peut comporter un seul pigment interférentiel multicouche ou plusieurs pigments interférentiels multicouche ayant des longueurs d'onde dominantes différentes.

Le pigment interférentiel multicouche peut comporter un substrat (encore appelé coeur ou noyau) recouvert sur au moins une face d'une ou plusieurs couches en des matériaux et épaisseurs choisis de telle sorte qu'une couleur soit produite par interférences.

Des couches du pigment interférentiel peuvent entourer ou non le substrat, lequel peut présenter une forme aplatie ou non.

Le substrat peut comporter du mica naturel, du mica synthétique, du verre, de l'alumine, de la silice, ou encore un quelconque métal, alliage ou oxyde métallique. La nature du substrat pourra être choisie en fonction de la brillance souhaitée. Par exemple, pour un rendu très brillant, un substrat en verre ou en métal pourra être préféré.

Le pigment interférentiel peut comporter plus de quatre couches d'indices de réfraction différents.

La taille des particules du pigment interférentiel multicouche, donnée par la granulométrie moyenne à la moitié de la population, encore appelée $D_{50}$, va par exemple de 1 μm à 2000 μm, mieux de 5 μm à 2000μm.

La proportion de pigment interférentiel multicouche est par exemple supérieure à 7 % et va par exemple de 7 à 20 % pour une composition non pulvérulente, liquide ou coulée, par exemple en stick et de 40 à 95 % pour une composition pulvérulente, libre ou compactée.

La couvrance de la composition peut être due essentiellement à la teneur en pigment interférentiel multicouche. En variante, au moins un pigment diffusant et/ou des charges peuvent conférer de la couvrance.

Les nacres sont des exemples de pigments interférentiels multicouche pouvant convenir.

### Nacres

Par « nacre », il faut comprendre des particules colorées de toute forme, irisées ou non, notamment produites par certains mollusques dans leur coquille ou bien synthétisées et qui présentent un effet de couleur par interférence optique.

Comme exemples de nacres, on peut citer les pigments nacrés tels que le mica titane recouvert avec un oxyde de fer, le mica recouvert d'oxychlorure de bismuth, le mica titane recouvert avec de l'oxyde de chrome, le mica titane recouvert avec un colorant organique notamment ainsi que les pigments nacrés à base d'oxychlorure de bismuth.

Il peut également s'agir de particules de mica à la surface desquelles sont superposées au moins deux couches successives d'oxydes métalliques et/ou de matières colorantes organiques.

Les nacres peuvent présenter une couleur ou un reflet jaune, rose, rouge, bronze, orangé, brun, or et/ou cuivré.

A titre illustratif de nacres pouvant être introduites en tant que pigment interférentiel multicouche, on peut citer les nacres de couleur or notamment commercialisées par la société ENGELHARD sous le nom de Brillant gold 20 212G (Timica), Gold 222C (Cloisonne), Sparkle gold (Timica), Gold 4504 (Chromalite) et Monarch gold 233X (Cloisonne) ; les nacres bronzes notamment commercialisées par la société MERCK sous la dénomination Bronze fine (17384) (Colorona) et Bronze (17353) (Colorona) et par la société ENGELHARD sous la dénomination Super bronze (Cloisonne) ; les nacres oranges notamment commercialisées par la société ENGELHARD sous la dénomination Orange 363C (Cloisonne) et Orange MCR 101 (Cosmica) et par la société MERCK sous la dénomination Passion orange (Colorona) et Matte orange (17449) (Microna) ; les nacres de teinte brune notamment commercialisées par la société ENGELHARD sous la dénomination Nu-antique copper 340XB (Cloisonne) et Brown CL4509 (Chromalite) ; les nacres à reflet cuivre notamment commercialisées par la société ENGELHARD sous la dénomination Copper 340A (Timica) ; les nacres à reflet rouge notamment commercialisées par la société MERCK sous la dénomination Sienna fine (17386) (Colorona) ; les nacres à reflet jaune notamment commercialisées par la société ENGELHARD sous la dénomination Yellow (4502) (Chromalite) ; les nacres de teinte rouge à reflet or notamment commercialisées par la société ENGELHARD sous la dénomination Sunstone G012 (Gemtone) ; les nacres roses notamment commercialisées par la société ENGELHARD sous la dénomination Tan opale G005 (Gemtone) ; les nacres noires à reflet or notamment commercialisées par la société ENGELHARD sous la dénomination Nu antique bronze 240 AB (Timica), les nacres bleues notamment commercialisées par la société MERCK sous la dénomination Matte blue (17433) (Microna), les nacres blanches à reflet argenté notamment commercialisées par la société MERCK sous la dénomination Xirona Silver et les nacres orangées rosées vert doré notamment commercialisées par la société MERCK sous la dénomination Indian summer (Xirona) et leurs mélanges.

Des pigments interférentiels multicouche présentant des propriétés magnétiques sont par exemple ceux commercialisés sous les dénominations commerciales COLORONA BLACKSTAR BLUE, COLORONA BLACKSTAR GREEN, COLORONA BLACKSTAR GOLD, COLORONA BLACKSTAR RED, CLOISONNE NU ANTIQUE SUPER GREEN, MICRONA MATTE BLACK (17437), MICA BLACK (17260), COLORONA PATINA SILVER (17289) et COLORONA PATINA GOLD (117288) de la société MERCK ou bien encore FLAMENCO TWILIGHT RED, FLAMENCO TWILIGHT GREEN, FLAMENCO TWILIGHT GOLD, FLAMENCO TWILIGHT BLUE, TIMICA NU ANTIQUE SILVER 110 AB, TIMICA NU ANTIQUE GOLD 212 GB, TIMICA NU-ANTIQUE COPPER 340 AB, TIMICA NU ANTIQUE BRONZE 240 AB, CLOISONNE NU ANTIQUE GREEN 828 CB, CLOISONNE NU ANTIQUE BLUE 626 CB, GEMTONE MOONSTONE G 004, CLOISONNE NU ANTIQUE RED 424 CB, CHROMA-LITE BLACK (4498), CLOISONNE NU ANTIQUE ROUGE FLAMBE (code 440 XB), CLOISONNE NU ANTIQUE BRONZE (240 XB), CLOISONNE NU ANTIQUE GOLD (222 CB) et CLOISONNE NU ANTIQUE COPPER (340 XB) de la société ENGELHARD.

Le pigment interférentiel de chacune des compositions de la gamme peut avantageusement être choisi parmi ceux conférant une couleur blanche dans la masse à la composition, à savoir les nacres notamment commercialisées par la société ENGELHARD sous le nom de SPARKLE 110P (Timica), Flamenco blue (Flamenco), Flamenco green (Flamenco), Flamenco red (Flamenco), Flamenco violet (Flamenco), Flamenco orange (Flamenco), Silkbalnc 110W (Timica), Extra large sparkle (Timica), Flamenco sparkle Gold (Flamenco), Flamenco sparkle green (Flamenco), Flamenco sparkle orange (Flamenco), Flamenco sparkle blue (Flamenco), Flamenco sparkle violet (Flamenco), Flamenco sparkle red (Flamenco), Flamenco summit gold (Flamenco) ; les nacres commercialisées par la société MERCK sous la dénomination Silk blue Timiron ($Mica/TiO_2/SnO_2$), Silk green Timiron ($Mica/TiO_2/SnO_2$), Silk red Timiron ($Mica/TiO_2/SnO_2$), Super red Timiron ($Mica/TiO_2$), Super green Timiron ($Mica/TiO_2$), Super blue Timiron ($Mica/TiO_2$), Artic Silver Timiron, Splendid copper Timiron ($Mica/TiO_2/SiO_2$), Splendid Violet Timiron ($Mica/TiO_2/SiO_2$) ; les nacres notamment commercialisées par la société ECKART sous la dénomination Prestige Silver (Prestige), Prestige Silver Star (Prestige), Prestige Gold (Prestige), Prestige soft gold (Prestige), Prestige silk green (Prestige), Prestige silk lilac (Prestige), Prestige silk blue (Prestige), Prestige silk red (Prestige).

Le pigment interférentiel peut être dépourvu de couche d'un matériau coloré, notamment choisi parmi FeOOH, $Fe_2O_3$, $Cr_2O_3$, $TiO_{2-x}$, $TiO_xN_y$, $CrPO_4$, $KFe[Fe(CN)_6]$, $Fe_3O_4$, TiO, TiN, $FeTiO_3$, C, Ag, Au, Fe, Mo, Cr, W.

Le pigment interférentiel peut par exemple ne comporter sur le substrat qu'une ou plusieurs couches des matériaux choisis parmi $TiO_2$ (rutile ou anatase), $ZrO_2$, $SnO_2$, $SiO_2$.

Le pigment interférentiel multicouche peut encore être choisi parmi les particules réfléchissantes interférentielles.

Particules réfléchissantes interférentielles

Ces particules peuvent être choisies parmi les particules à substrat synthétique enrobé au moins partiellement d'au moins une couche d'au moins un oxyde métallique, choisi par exemple parmi les oxydes de titane, notamment $TiO_2$, de fer notamment $Fe_2O_3$, d'étain, de chrome, le sulfate de baryum et les matériaux suivants : $MgF_2$, $CrF_3$, ZnS, ZnSe, $SiO_2$, $Al_2O_3$, MgO, $Y_2O_3$, $SeO_3$, SiO, $HfO_2$, $ZrO_2$, $CeO_2$, $Nb_2O_5$, $Ta_2O_5$, $MoS_2$ et leurs mélanges ou alliages.

A titre d'exemple de telles particules, on peut citer par exemple les particules comportant un substrat de mica synthétique revêtu de dioxyde de titane, ou les particules de verre enrobé soit d'oxyde de fer brun, d'oxyde de titane, d'oxyde d'étain ou d'un de leurs mélanges comme celles vendues sous la marque REFLECKS[®] par la société ENGELHARD.

Le pigment interférentiel multicouche peut encore être un pigment goniochromatique.

Pigment goniochromatique

Par « pigment goniochromatique », on désigne au sens de la présente invention un pigment permettant d'obtenir, lorsque la composition est étalée sur un support, un trajet de couleur dans le plan a*b* de l'espace colorimétrique CIE 1976 qui correspond à une variation Dh° de l'angle de teinte h° d'au moins 20° lorsque l'on fait varier l'angle d'observation par rapport à la normale entre 0° et 80°, pour un angle d'incidence de la lumière de 45°.

Le trajet de couleur peut être mesuré par exemple au moyen d'un spectrogonioréflectomètre de marque INSTRUMENT SYSTEMS et de référence GON 360 GONIOMETER, après que la composition a été étalée à l'état fluide avec une épaisseur de 300 μm au moyen d'un étaleur automatique sur une carte de contraste de marque ERICHSEN et de référence Typ 24/5, la mesure étant effectuée sur le fond noir de la carte.

Le pigment goniochromatique peut être choisi par exemple parmi les structures multicouches interférentielles et les agents de coloration à cristaux liquides.

Dans le cas d'une structure multicouche, celle-ci peut comporter par exemple au moins deux couches, chaque couche étant réalisée par exemple à partir d'au moins un matériau choisi dans le groupe constitué par les matériaux suivants : $MgF_2$, $CeF_3$, $ZnS$, $ZnSe$, $Si$, $SiO_2$, $Ge$, $Te$, $Fe_2O_3$, $Pt$, $Va$, $Al_2O_3$, $MgO$, $Y_2O_3$, $S_2O_3$, $SiO$, $HfO_2$, $ZrO_2$, $CeO_2$, $Nb_2O_5$, $Ta_2O_5$, $TiO_2$, $Ag$, $Al$, $Au$, $Cu$, $Rb$, $Ti$, $Ta$, $W$, $Zn$, $MoS_2$, cryolithe, alliages, polymères et leurs associations.

La structure multicouche peut présenter ou non, par rapport à une couche centrale, une symétrie au niveau de la nature chimique des couches empilées.

Selon l'épaisseur et la nature des différentes couches, on obtient différents effets.

Des exemples de structures multicouche interférentielles symétriques sont par exemple les structures suivantes : $Fe_2O_3/SiO_2/Fe_2O_3/SiO_2/Fe_2O_3$, un pigment ayant cette structure étant commercialisé sous la dénomination SICOPEARL par la société BASF ; $MoS_2/SiO_2/mica$-oxyde$/SiO_2/MoS_2$ ; $Fe_2O_3/SiO_2/mica$-oxyde$/SiO_2/Fe_2O_3$ ; $TiO_2/SiO_2/TiO_2$ et $TiO_2/Al_2O_3/TiO_2$, des pigments ayant ces structures étant commercialisés sous la dénomination XIRONA par la société MERCK (Darmstadt).

Les agents de coloration à cristaux liquides comprennent par exemple des silicones ou des éthers de cellulose sur lesquels sont greffés des groupes mésomorphes. Comme particules goniochromatiques à cristaux liquides, on peut utiliser par exemple celles vendues par la société CHENIX ainsi que celles commercialisées sous la dénomination HELICONE® HC par la société WACKER.

Comme pigment goniochromatique, on peut encore utiliser certaines nacres, des pigments à effets sur substrat synthétique, notamment substrat type alumine, silice, borosilicate, oxyde de fer, aluminium, ou des paillettes interférentielles issues d'un film de polytéréphthalate.

Le matériau peut en outre comporter des fibres goniochromatiques dispersées. De telles fibres pourront présenter une longueur inférieure à 80 μm par exemple.

**Milieu cosmétiquement acceptable**

Par « milieu cosmétiquement acceptable », on désigne un milieu non toxique susceptible d'être appliqué sur les matières kératiniques d'êtres humains.

Le milieu cosmétiquement acceptable sera adapté à la nature du support sur lequel doit être appliqué la composition ainsi qu'à la forme sous laquelle la composition est destinée à être conditionnée.

La composition selon l'invention peut comprendre un milieu aqueux et/ou une phase grasse, être anhydre ou non.

Phase aqueuse ou grasse

La composition peut comprendre de l'eau ou un mélange d'eau et de solvants organiques hydrophiles comme les alcools et notamment des monoalcools inférieurs linéaires ou ramifiés ayant de 2 à 5 atomes de carbone comme l'éthanol, l'isopropanol ou le n-propanol, les polyols comme la glycérine, la diglycérine, le propylène glycol, le sorbitol, le penthylène glycol, les polyéthylène glycols.

La phase hydrophile peut, en outre, contenir des éthers en $C_2$ et des aldéhydes en $C_2$-$C_4$ hydrophiles.

L'eau ou le mélange d'eau et de solvants organiques hydrophiles peut être présent dans la composition selon l'invention en une teneur allant de 0 % à 90 %, notamment 0,1 % à 90 % en poids, par rapport au poids total de la composition, et de préférence de 0 % à 60 % en poids, notamment 0,1 % à 60 % en poids.

La composition peut également comprendre une phase grasse, notamment constituée de corps gras liquides à 25 °C et éventuellement de corps gras solides à température ambiante tels que les cires, les corps gras pâteux, les gommes et leurs mélanges.

Comme corps gras liquides à température ambiante, appelés souvent huiles, utilisables dans l'invention, on peut citer entre autres les huiles hydrocarbonées végétales telles que les triglycérides liquides d'acides gras de 4 à 10 atomes de carbone comme les triglycérides des acides heptanoïque ou octanoïque, ou encore les huiles de tournesol, de maïs, de soja, de pépins de raisin, de sésame, d'abricot, de macadamia, de ricin, d'avocat, les triglycérides des acides caprylique/caprique, l'huile de jojoba, de beurre de karité ; les hydrocarbures linéaires ou ramifiés, d'origine minérale ou synthétique tels que les huiles de paraffine et, notamment les isoparaffines en $C_8$-$C_{16}$ telles que l'isododé-

cane, l'isodécane, l'isohexadécane, la vaseline, les polydécènes, le polyisobutène hydrogéné tel que le Parléam®, le squalane ; les esters et les éthers de synthèse notamment d'acides gras comme par exemple l'huile de Purcellin, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras ; des esters de polyol comme le dioctanoate de propylène glycol, le diheptanoate de néopentylglycol, le diisononanoate de diéthylèneglycol ; et les esters du pentaérythritol ; des alcools gras ayant de 12 à 26 atomes de carbone comme l'octyldodécanol, le 2-butyloctanol, le 2-hexyldécanol, le 2-undécylpentadécanol, l'alcool oléique ; les huiles fluorées partiellement hydrocarbonées et/ou siliconées ; les huiles siliconées comme les polyméthylsiloxanes (PDMS) volatiles ou non, linéaires ou cycliques, liquides ou pâteux à température ambiante comme les cyclométhicones, les diméthicones, comportant éventuellement un groupement phényle, comme les phényl triméthicones, les phényltriméthylsiloxydiphényl siloxanes, les diphénylméthyldiméthyl-trisiloxanes, les diphényl diméthicones, les phényl diméthicones, les polyméthylphényl siloxanes ; leurs mélanges.

Ces huiles peuvent être présentes en une teneur allant de 0,01 à 90 % par rapport au poids total de la composition.

La composition selon l'invention peut également comprendre un ou plusieurs solvants organiques, physiologiquement acceptables. Ce ou ces solvants, qui peuvent être lipophiles, peuvent être présents en une teneur allant de 0 à 90 %, mieux de 0 à 60% en poids, par rapport au poids total de la composition et encore mieux de 0,1 à 30%.

Le milieu peut comporter une phase organique liquide dans laquelle de l'eau est dispersée ou émulsionnée.

La composition peut encore présenter une phase grasse continue, pouvant contenir moins de 5% d'eau, notamment moins de 1% d'eau par rapport à son poids total et en particulier être sous forme anhydre.

Agent filmogène

Le milieu peut comporter un agent filmogène, notamment un polymère filmogène.

On entend par « agent filmogène » un agent apte à former à lui seul ou en présence d'un agent auxiliaire de filmification, un film macroscopiquement continu et adhérent sur les matières kératiniques, et de préférence un film cohésif, et mieux encore un film dont la cohésion et les propriétés mécaniques sont telles que ledit film peut être isolable et manipulable isolément, par exemple lorsque ledit film est réalisé par coulage sur une surface antiadhérente comme une surface téflonnée ou siliconnée.

La composition peut comporter une phase aqueuse et le polymère filmogène peut être présent dans cette phase aqueuse. Celui-ci pourra être un polymère en dispersion ou en solution.

La composition peut comporter une phase huileuse et le polymère filmogène peut être présent dans cette phase huileuse. Le polymère pourra alors être en dispersion ou en solution.

Parmi les polymères filmogènes utilisables, on peut citer les polymères synthétiques, de type radicalaire ou de type polycondensat, les polymères d'origine naturelle, et leurs mélanges.

Les polymères filmogènes de type radicalaire peuvent être notamment des polymères, ou des copolymères, vinyliques, notamment des polymères acryliques.

Parmi les polycondensats filmogènes, on peut citer les polyuréthanes, les polyesters, les polyesters amides, les polyamides, et les résines époxyesters, les polyurées.

Les polyesters peuvent être obtenus, de façon connue, par polycondensation d'acides dicarboxyliques avec des polyols, notamment des diols.

Les polyesters amides peuvent être obtenus de manière analogue aux polyesters, par polycondensation de diacides avec des diamines ou des amino alcools.

A titre d'exemple de polymères filmogènes liposolubles, on peut citer les copolymères d'ester vinylique (le groupe vinylique étant directement relié à l'atome d'oxygène du groupe ester et l'ester vinylique ayant un radical hydrocarboné saturé, linéaire ou ramifié, de 1 à 19 atomes de carbone, lié au carbonyle du groupe ester) et d'au moins un autre monomère qui peut être un ester vinylique (différent de l'ester vinylique déjà présent), une α-oléfine (ayant de 8 à 28 atomes de carbone), un alkylvinyléther (dont le groupe alkyl comporte de 2 à 18 atomes de carbone), ou un ester allylique ou méthallylique (ayant un radical hydrocarboné saturé, linéaire ou ramifié, de 1 à 19 atomes de carbone, lié au carbonyle du groupe ester).

Ces copolymères peuvent être réticulés à l'aide de réticulants qui peuvent être soit du type vinylique, soit du type allylique ou méthallylique, tels que le tétraallyloxyéthane, le divinylbenzène, l'octanedioate de divinyle, le dodécanedioate de divinyle, et l'octadécanedioate de divinyle.

Comme exemples de ces copolymères, on peut citer les copolymères : acétate de vinyle/stéarate d'allyle, l'acétate de vinyle/laurate de vinyle, acétate de vinyle/stéarate de vinyle, acétate de vinyle/octadécène, acétate de vinyle/octadécylvinyléther, propionate de vinyle/laurate d'allyle, propionate de vinyle/laurate de vinyle, stéarate de vinyle/octadécène-1, acétate de vinyle/dodécène-1, stéarate de vinyle/éthylvinyléther, propionate de vinyle/cétyl vinyle éther, stéarate de vinyle/acétate d'allyle, diméthyl-2, 2 octanoate de vinyle/laurate de vinyle, diméthyl-2, 2 pentanoate d'allyle/ laurate de vinyle, diméthyl propionate de vinyle/stéarate de vinyle, diméthyl propionate d'allyle/stéarate de vinyle, propionate de vinyle/stéarate de vinyle, réticulé avec 0,2 % de divinyl benzène, diméthyl propionate de vinyle/laurate de vinyle, réticulé avec 0,2 % de divinyl benzène, acétate de vinyle/octadécyl vinyl éther, réticulé avec 0,2 % de tétraallyloxyéthane, acétate de vinyle/stéarate d'allyle, réticulé avec 0,2 % de divinyl benzène, acétate de vinyle/octadécène-1 réticulé avec 0,2 % de divinyl benzène et propionate d'allyle/stéarate d'allyle réticulé avec 0,2 % de divinyl benzène.

Le polymère filmogène peut encore être choisi parmi les résines de silicone, généralement solubles ou gonflables dans les huiles de silicone, qui sont des polymères de polyorganosiloxanes réticulés.

Le polymère filmogène peut être également présent dans la composition sous la forme de particules en dispersion dans une phase aqueuse ou dans une phase solvant non aqueuse, connue généralement sous le nom de latex ou pseudolatex. Les techniques de préparation de ces dispersions sont bien connues de l'homme du métier.

La composition selon l'invention peut comprendre un agent plastifiant favorisant la formation d'un film avec le polymère filmogène. Un tel agent plastifiant peut être choisi parmi tous les composés connus de l'homme du métier comme étant susceptibles de remplir la fonction recherchée.

Bien entendu, cette liste de polymères n'est pas exhaustive.

Autres agents de coloration

La composition peut comporter un ou plusieurs pigments diffusants, générant un couleur par un phénomène d'absorption, dans une proportion permettant de conserver le phénomène d'interférences responsable de la couleur de la composition une fois appliquée sur les matières kératiniques.

La composition peut ne pas comporter d'autres agents de coloration que le ou les pigment(s) interférentiel(s) multicouche, notamment être dépourvue de pigments à base d'oxydes de fer ou autres pigments générant une couleur par un phénomène d'absorption.

Chaque composition de la gamme peut par exemple contenir moins de 0,5% en masse de pigments générant une couleur par absorption, notamment moins de 0,5% de pigments à base d'oxydes de fer, mieux moins de 0,2%.

Lorsque des pigments diffusants sont présents, divers pigments diffusants peuvent être envisagés, étant par exemple choisi parmi les laques ou pigments organiques sélectionnés notamment parmi les matériaux ci-dessous et leurs mélanges :

- le carmin de cochenille,
- les pigments organiques de colorants azoïques, anthraquinoniques, indigoïdes, xanthéniques, pyréniques, quinoliniques, de triphénylméthane, de fluorane,
- les laques organiques ou sels insolubles de sodium, de potassium, de calcium, de baryum, d'aluminium, de zirconium, de strontium, de titane, de colorants acides tels que les colorants azoïques, anthraquinoniques, indigoïdes, xanthéniques, pyréniques, quinoliniques, de triphénylméthane, de fluorane, ces colorants pouvant comporter au moins un groupe acide carboxylique ou sulfonique.

Parmi les pigments organiques, on peut notamment citer ceux connus sous les dénominations suivantes : D&C Blue n° 4, D&C Brown n° 1, D&C Green n° 5, D&C Green n° 6, D&C Orange n° 4, D&C Orange n° 5, D&C Orange n° 10, D&C Orange n° 11, D&C Red n° 6, D&C Red n° 7, D&C Red n° 17, D&C Red n° 21, D&C Red n° 22, D&C Red n° 27, D&C Red n° 28, D&C Red n° 30, D&C Red n° 31, D&C Red n° 33, D&C Red n° 34, D&C Red n° 36, D&C Violet n° 2, D&C Yellow n° 7, D&C Yellow n° 8, D&C Yellow n° 10, D&C Yellow n° 11, FD&C Blue n° 1, FD&C Green n° 3, FD&C Red n° 40, FD&C Yellow n° 5, FD&C Yellow n° 6.

La laque peut être supportée par un support organique tel que la colophane ou le benzoate d'aluminium, par exemple.

Parmi les laques organiques, on peut en particulier citer celles connues sous les dénominations suivantes : D&C Red n° 2 Aluminium lake, D&C Red n° 3 Aluminium lake, D&C Red n° 4 Aluminium lake, D&C Red n° 6 Aluminium lake, D&C Red n° 6 Barium lake, D&C Red n° 6 Barium/Strontium lake, D&C Red n° 6 Strontium lake, D&C Red n° 6 Potassium lake, D&C Red n° 7 Aluminium lake, D&C Red n° 7 Barium lake, D&C Red n° 7 Calcium lake, D&C Red n° 7 Calcium/Strontium lake, D&C Red n° 7 Zirconium lake, D&C Red n° 8 Sodium lake, D&C Red n° 9 Aluminium lake, D&C Red n° 9 Barium lake, D&C Red n° 9 Barium/Strontium lake, D&C Red n° 9 Zirconium lake, D&C Red n° 10 Sodium lake, D&C Red n° 19 Aluminium lake, D&C Red n° 19 Barium lake, D&C Red n° 19 Zirconium lake, D&C Red n° 21 Aluminium lake, D&C Red n° 21 Zirconium lake, D&C Red n° 22 Aluminium lake, D&C Red n° 27 Aluminium lake, D&C Red n° 27 Aluminium/Titanium/Zirconium lake, D&C Red n° 27 Barium lake, D&C Red n° 27 Calcium lake, D&C Red n° 27 Zirconium lake, D&C Red n° 28 Aluminium lake, D&C Red n° 30 lake, D&C Red n° 31 Calcium lake, D&C Red n° 33 Aluminium lake, D&C Red n° 34 Calcium lake, D&C Red n° 36 lake, D&C Red n° 40 Aluminium lake, D&C Blue n° 1 Aluminium lake, D&C Green n° 3 Aluminium lake, D&C Orange n° 4 Aluminium lake, D&C Orange n° 5 Aluminium lake, D&C Orange n° 5 Zirconium lake, D&C Orange n° 10 Aluminium lake, D&C Orange n° 17 Barium lake, D&C Yellow n° 5 Aluminium lake, D&C Yellow n° 5 Zirconium lake, D&C Yellow n° 6 Aluminium lake, D&C Yellow n° 7 Zirconium lake, D&C Yellow n° 10 Aluminium lake, FD&C Blue n° 1 Aluminium lake, FD&C Red n° 4 Aluminium lake, FD&C Red n° 40 Aluminium lake, FD&C Yellow n° 5 Aluminium lake, FD&C Yellow n° 6 Aluminium lake.

Les matériaux chimiques correspondant à chacune des matières colorantes organiques citées précédemment sont mentionnés dans l'ouvrage « International Cosmetic Ingredient Dictionnary and Handbook », Edition 1997, pages 371 à 386 et 524 à 528, publié par « The Cosmetic, Toiletry, and Fragrance Association », dont le contenu est incorporé dans la présente demande par référence.

Le pigment diffusant peut être un pigment composite, comportant un noyau enrobé au moins partiellement par une écorce. Un tel pigment composite peut être composé notamment de particules comportant un noyau inorganique et au moins un enrobage au moins partiel d'au moins une matière colorante organique. Au moins un liant peut avantageusement contribuer à la fixation de la matière colorante organique sur le noyau inorganique.

Les particules de pigment composite peuvent présenter des formes variées. Ces particules peuvent être notamment en forme de plaquettes ou globulaires, en particulier sphériques, et être creuses ou pleines. Par « en forme de plaquettes », on désigne des particules dont le rapport de la plus grande dimension à l'épaisseur est supérieur ou égal à 5. Un pigment composite peut présenter par exemple une surface spécifique comprise entre 1 et 1000 m$^2$/g, notamment entre 10 et 600 m$^2$/g environ, et en particulier entre 20 et 400 m$^2$/g environ. La surface spécifique est la valeur mesurée par la méthode BET. La proportion massique du noyau peut excéder 50 % par rapport au poids total du pigment composite, par exemple aller de 50 % à 70 %, par exemple de 60 % à 70 %.

L'agent de coloration peut encore être un colorant.

Il peut s'agir d'un colorant d'origine végétale, animale ou minérale, en particulier d'origine végétale ou minérale, notamment d'origine végétale. Ce colorant peut être de nature non synthétique.

Le colorant peut être un colorant naturel hydrosoluble ou liposoluble.

A titre illustratif des agents de coloration naturels hydrosolubles susceptibles d'être mis en oeuvre selon l'invention, on peut particulièrement citer le caramel, le jus de betterave et le carmin, la bétanine (betterave), la chlorophylline cuivrée, le bleu de méthylène, les anthocyanines (enocianine, carotte noire, hibiscus, sureau) et la riboflavine.

A titre illustratif des agents de coloration naturels liposolubles susceptibles d'être mis en oeuvre selon l'invention, on peut particulièrement citer le rouge Soudan, le β-carotène, les caroténoïdes, le lycopène, l'huile de palme, le brun Soudan, le jaune quinoléique les xanthophylles (capsanthine, capsorubine, lutéine), et le curcumin.

Comme autres colorants naturels, on peut plus particulièrement citer les anthocyanes de fleurs ou de fruits ou leurs dérivés, les flavonoïdes et tanins extraits de végétaux natifs ou fermentés, la juglone, la lawsone, les extraits de soja fermenté, d'algues, de champignons, de micro-organismes, les sels de Flavylium non substitués en position 3 tels que décrit dans le brevet EP 1 172 091, les extraits de Gesneria Fulgens, Blechum Procerum, Saxifraga et les pigments susceptibles d'être obtenus par extraction par un solvant organique ou hydro-organique d'un milieu de culture de micromycètes du type monascus Monascus.

Comme exemple de colorants synthétiques, on peut citer les colorants liposolubles synthétiques tels que, par exemple, le DC Red 17, le DC Red 21, le DC Red 27, le DC Green 6, le DC Yellow 11, le DC Violet 2 et le DC Orange 5.

Comme exemple de colorants hydrosolubles synthétiques, on peut citer le FDC Red 4, le DC Red 6, le DC Red 22, le DC Red 28, le DC Red 30, le DC Red 33, le DC Orange 4, le DC Yellow 5, le DC Yellow 6, le DC Yellow 8, le FDC Green 3, le DC Green 5 et le FDC Blue 1.

Charges

La composition cosmétique peut comprendre des charges.

Par « charges », on désigne des particules de toute forme insolubles dans le milieu de la composition, quelle que soit la température à laquelle la composition est fabriquée. Ces charges peuvent servir notamment à modifier la rhéologie ou la texture de la composition.

A titre d'exemple de charges, on peut citer, entre autres, le talc, le mica, la silice, le kaolin, et les poudres de polyamide (Nylon® ou Orgasol® de chez Atochem).

La teneur en charges sera choisie de façon à ne pas entraver outre mesure le résultat recherché.

Actifs et autres composés

La composition cosmétique peut également contenir un ou plusieurs actifs cosmétiques, dermatologiques, hygiéniques ou pharmaceutiques.

Comme actifs cosmétiques, dermatologiques, hygiéniques ou pharmaceutiques, utilisables dans les compositions de l'invention, on peut citer les hydratants (polyol comme glycérine), vitamines (C, A, E, F, B, ou PP), acides gras essentiels, huiles essentielles, céramides, sphingolipides, filtres solaires liposolubles ou sous forme de nano-particules, les actifs spécifiques de traitement de la peau (agents de protection, anti-bactériens, antirides...). Ces actifs peuvent être utilisés par exemple à des concentrations de 0,001 à 15 % par rapport au poids total de la composition.

La composition cosmétique peut également contenir des ingrédients couramment utilisés en cosmétique, tels que par exemple les épaississants, les tensioactifs, les oligo-éléments, les hydratants, les adoucissants, les séquestrants, les parfums, les agents alcalinisants ou acidifiants, les conservateurs, les antioxydants, les filtres UV, ou leurs mélanges.

La composition cosmétique peut, de plus, comprendre, selon le type d'application envisagée, les constituants classiquement utilisés dans les domaines considérés, qui sont présents en une quantité appropriée à la forme galénique souhaitée.

**Formes galéniques**

Chaque composition cosmétique de la gamme peut se présenter sous toute forme galénique normalement utilisée pour une application topique et notamment sous forme anhydre ou non anhydre, sous forme solide, semi-solide, d'une solution huileuse ou aqueuse, d'un gel huileux ou aqueux, d'une émulsion huile-dans-eau ou eau-dans-huile, d'une émulsion multiple, d'une dispersion d'huile dans de l'eau grâce à des vésicules situés à l'interface huile/eau ou d'un spray.

De préférence, toutes les compositions de la gamme se présentent de la même forme galénique.

Par « composition anhydre », on désigne une composition ayant moins de 5 % en masse d'eau, mieux moins de 3 %, voire moins de 1 %. Une composition anhydre peut ne pas comporter d'eau ajoutée intentionnellement au cours de la préparation de la composition.

Chaque de la gamme composition peut notamment se présenter sous forme de stick.

Les compositions cosmétiques de la gamme peuvent constituer chacune, entre autres produits de maquillage, un rouge à lèvres, un gloss liquide, une pâte de rouge à lèvres, un fard à joues, un fond de teint, un produit de contour des yeux, un eye-liner, un mascara, un vernis à ongles, une ombre à paupières, une base de maquillage, un produit de maquillage du corps ou des cheveux.

Toutes les compositions de la gamme peuvent être destinées au maquillage de la peau, des lèvres, des ongles, des cils ou sourcils ou des cheveux.

Toutes les compositions de la gamme peuvent être obtenues selon les procédés de préparation classiquement utilisés en cosmétique.

**Conditionnement et modes d'application**

Chaque composition de la gamme peut être conditionnée dans tout réceptacle ou sur tout support prévu à cet effet.

Chaque composition peut être appliquée en utilisant un applicateur, floqué ou non, par exemple une mousse, un embout, un pinceau, un feutre, une spatule, un fritté, une brosse, un peigne, un tissé ou non tissé.

L'application peut encore s'effectuer avec le doigt ou en déposant directement la composition sur le support à maquiller, par exemple par friction dans le cas d'un stick ou par pulvérisation ou projection à l'aide d'un dispositif piézoélectrique ou par transfert d'une couche de composition préalablement déposée sur un support intermédiaire.

Chaque composition peut, le cas échéant, être appliquée comme couche de base (base coat) recouverte d'une couche d'une autre composition (top coat), destinée par exemple à conférer de la brillance, ou comme couche de revêtement sur une couche d'une autre composition, voire entre une couche de base et une couche de revêtement.

Chaque composition peut être conditionnée dans un dispositif de conditionnement qui laisse apparaître la couleur de la composition dans la masse.

Il peut s'agir d'un récipient ayant un corps au moins partiellement transparent et/ou d'un dispositif de conditionnement comportant un organe de bouchage au moins partiellement transparent.

Chaque composition peut être conditionnée dans un dispositif qui permet de voir à la fois la couleur de la composition dans la masse et la couleur de la composition après application sur des matières kératiniques.

Chaque composition peut encore être conditionnée dans un dispositif qui comporte des moyens représentatifs de la couleur révélée à l'application, par exemple un dépôt d'une couche de la composition ou d'une encre ou vernis comportant les mêmes agents de coloration que la composition.

Le cas échéant, chaque composition peut être conditionnée avec un aimant permettant de modifier l'orientation des particules de pigment interférentiel multicouche, lorsque ce pigment présente une susceptibilité magnétique non nulle.

**Procédé de présentation et présentoir**

L'invention a encore pour objet, selon un autre de ses aspects, un procédé de présentation d'une gamme de compositions cosmétiques conformes à l'invention, comportant les étapes consistant à :

- éclairer au moins une première région d'un échantillon de la gamme avec un éclairage diffus de façon à faire apparaître sa couleur dans la masse,
- éclairer au moins une deuxième région d'un échantillon de la gamme avec un éclairage directionnel de façon à faire apparaître la couleur interférentielle.

Les première et deuxième régions peuvent être des régions différentes d'un même échantillon de composition.

En variante, les première et deuxième régions appartiennent à des échantillons différents.

En variante encore, les première et deuxième régions correspondent à une même région et les éclairages diffus et directionnel sont appliqués alternativement.

L'invention a encore pour objet un procédé de présentation dans lequel :

- on éclaire deux compositions de la gamme de façon à faire apparaître une même couleur dans la masse,
- on éclaire ces deux compositions de manière à révéler des couleurs différentes après application, les pigments interférentiels multicouche de ces compositions étant différents.

L'invention a encore pour objet, selon un autre de ses aspects, un présentoir comportant :

- une source d'éclairage directionnel,
- une source d'éclairage diffus,
- un support pour permettre d'exposer au moins un échantillon de la gamme, mieux deux échantillons provenant de compositions différentes de la gamme, à la source d'éclairage diffus et au moins un échantillon, mieux les deux échantillons, à la source d'éclairage directionnel.

**Procédé de maquillage**

L'invention a encore pour objet un procédé de maquillage des matières kératiniques au moyen d'une composition d'une gamme selon l'invention.

Il peut s'agir du maquillage de la peau, des lèvres, des ongles, des cils, sourcils ou cheveux.

**Kit**

La présente invention a également pour objet un kit de maquillage comportant :

- une première composition d'une gamme selon l'invention,
- une deuxième composition comportant un milieu cosmétiquement acceptable et à appliquer sous ou sur la première composition.

Cette deuxième composition est par exemple destinée à améliorer la tenue de la première composition et/ou à en modifier l'aspect.

**Exemples proposés**

Exemple 1 : Gamme de quatre brillants à lèvres

| Octyl-2 dodécanol | 10 |
|---|---|
| Ditertiobutyl 4-hydroxytoluène | 0,07 |
| Polybutène (monooléfines / isoparaffines 95/5) (PM : 2060) | 37 |
| Mélange de p-hydroxybenzoates d'isopropyle, iso-butyle, n-butyle (40/30/30) | 0,6 |
| Tetra-iso-stéarate de pentaérythrityle | 11,33 |
| Tri-méllitate de tridécyle | 11 |

(suite)

| | |
|---|---|
| Triglycéride d'acide 2-décyl tétradécanoïque (GUERBET C24) | 15 |
| Pigment interférentiel multicouche Pk* | 15 |

* P$_1$ : XIRONA RED de la société MERCK pour la première composition de la gamme.
P$_2$ : XIRONA BLUE de la société MERCK pour la deuxième composition de la gamme.
P$_3$ : XIRONA GREEN de la société MERCK pour la troisième composition de la gamme.
P$_4$ : XIRONA SILVER de la société MERCK pour la quatrième composition de la gamme.

Exemple 2 : Gamme de trois rouges à lèvres

| | |
|---|---|
| Tri-méllitate de tri-décyle | 11 |
| Lanoline liquide | $Y_k$ |
| Malate de d'iso-stéaryle | 13 |
| Lanoline acétylée | **10** |
| Triglycérides d'acides laurique/palmitique/cétylique/stéarique (50/20/10/10) | 5 |
| Cire microcrystalline (C20-C60) | 3 |
| Lanolate d'isopropyle protégé | 10 |
| Octyl-2-décanol | $Z_k$ |
| Phényl triméthylsiloxy trisiloxane (VISCOSITE: 20 CST - PM: 372) | 4 |
| Cire de polyéthylène (PM :500) | 8 |
| Pigment interférentiel multicouche* | $X_k$ |
| *TIMIRON SILK RED de la société MERCK. | |

Les teneurs $X_k$ sont respectivement de $X_1$ = 13 ; $X_2$ = 7,2 et $X_3$ = 14,9 pour les trois compositions de la gamme.
Les teneurs $Y_k$ sont respectivement de $Y_1$ = 9 ; $Y_2$ = 10,5 et $Y_3$ = 8,5 pour les trois compositions de la gamme.
Les teneurs $Z_k$ sont respectivement de $Z_1$ = 14 ; $Z_2$ = 18,3 et $Z_3$ = 12,6 pour les trois compositions de la gamme.
On obtient les couvrances suivantes :

Composition 1 ($X_1$ = 13) couvrance : 41%
Composition 2 ($X_2$ = 7,2) couvrance : 32
Composition 3 ($X_3$ = 14,9) couvrance : 42,60

Exemple 3 : Gamme de trois fonds de teint liquide

| | |
|---|---|
| Triéthano lamine | |
| Acide éthylène diamine tetracétique, sel disodique, 2H2O | ,2 |
| Homopolymère carboxyvinylique réticulée | ,5 |
| Polyvinylpyrro lidone | ,6 |
| Glycérol | ,75 |
| Eau désionisée | 0,45 |
| 1,3 butylène glycol | |
| Microsphère de silice (3$\mu$m) | ,5 |

(suite)

| Pigment interférentiel multicouche $P_k$* | |
|---|---|
| $P_1$*:<br>Sicopearl fantastio ROSE X de la société BASF pour la première composition.<br>$P_2$: Sicopearl fantastio RUBIS X de la société BASF pour la deuxième composition.<br>$P_3$: Sicopearl fantastio VERT X de la société BASF pour la troisième composition. | |

Exemple 4 : Gamme de quatre vernis à ongles à base aqueuse

| | |
|---|---|
| Pyophosphate térasodique | ,2 |
| Polydiméhylsiloxane oxyéhylèe a terminaisons méthoxy | ,5 |
| Mélange de polyuréthane aliphatique, N-méthyl pyrrolidone, triéthylamine, eau (35/8,5/2/54,5) | 8 |
| Glycérol | |
| Eau désionisée | 5 |
| Alcool éthylique (96°) | ,8 |
| Laponite synthétique (silicate mixte magnésium/lithium/sodium) | ,3 |
| Pigment interférentiel multicouche $P_k$* | 1,2 |
| * $P_1$ : XIRONA RED de la société MERCK pour la première composition.<br>$P_2$ : XIRONA BLUE de la société MERCK pour la deuxième composition.<br>$P_3$ : XIRONA GREEN de la société MERCK pour la troisième composition.<br>$P_4$ : XIRONA SILVER de la société MERCK pour la quatrième composition de la gamme. | |

Exemple 5 : Gamme de quatre vernis à ongles à base solvant organique

| | |
|---|---|
| Nitrocellulose | 11 |
| N-éthyl o,p-toluènesulfonamide | 5 |
| Résine alkyde | 10 |
| Isopropanol | 4 |
| Pigment interférentiel multicouche $P_k$ * | 10 |
| Acétate de butyle/acétate d'éthyle 50/50 | Qsp 100 |
| * P1 : XIRONA RED de la société MERCK pour la première composition.<br>P2 : XIRONA BLUE de la société MERCK pour la deuxième composition.<br>P3 : XIRONA GREEN de la société MERCK pour la troisième composition.<br>P4 : XIRONA SILVER de la société MERCK pour la quatrième composition de la gamme. | |

Exemple 6 : Gamme de quatre fards à paupière en poudre

| | |
|---|---|
| TERTIOBUTYL 4-HYDROXYANISOLE | 0,012 |
| DITERTIOBUTYL 4-HYDROXYTOT.,UENE | 0,012 |
| VASELINE BLANCHE | 1,2 |
| ALCOOL OLEIQUE | 1,2 |
| LANOLINE LIQUIDE PROTEGEE | 0,66 |
| HUILE DE VASELINE | 6,516 |
| HUILE DE RICIN | 1,296 |
| MYRTSTATE D'ISOPROPYLE | 0,864 |
| P-HYDROXYBENZOATE DE PROPYLE | 0,24 |
| MELANGE P-HYDROXYBENZOATES DE METHYLE, ETHYLE, PROPYLE, BUTYLE ISOBUTYLE / PHENOXY -2 ETHANOL | 0,6 |
| Pigment intéreférentiel multicouche $P_k$ * | 50,3 |
| STEARATE DE MAGNESIUM | 4 |
| TALC | 30,1 |
| Pigment à réflexion métallique ** | 3 |
| * P1 : XIRONA RED de la société MERCK pour la première composition.<br>P2 : XIRONA BLUE de la société MERCK pour la deuxième composition.<br>P3 : XIRONA GREEN de la société MERCK pour la troisième composition.<br>P4 : XIRONA SILVER de la société MERCK pour la quatrième composition de la gamme.,<br>** METASHINE ME 2040 PS de la société NIPPON SHEET GLASS | |

Exemple 7 : Gamme de quatre fards à paupière liquides

| | |
|---|---|
| DITERTIOBUTYL 4-HYDROXYTOLUENE | ,09 |
| HECTORITE MODIFIEE DISTEARYL DIMETHYL AMMONIUM | ,74 |
| TRIGLYCERIDES D'ACIDES LAURIQUE/PALMITIQUE/CETYLIQUE/STEARIQUE (50/20/10/10) | ,46 |
| CARBONATE DE PROPYLENE | ,22 |
| CIRE D'ABEILLE BLANCHIE | ,77 |
| BEURRE DE SHOREA PROTEGE | ,7 |
| FRACTION LIQUIDE DE BEURRE DE KARITE PROTEGEE | ,85 |
| POUDRE DE NYLON 12 | 0,4 |
| ISO-DODECANE | 5,32 |
| P-HYDROXYBENZOATE DE PROPYLE | ,17 |
| PARAFFINE RAFFINEE PROTEGEE | ,88 |
| TALC | 0,4 |
| Pigment interférentiel multicouche* $P_k$ | 5 |

(suite)

| Pigment à réflexion métallique ** | |
| --- | --- |

* P₁ : XIRONA RED de la société MERCK pour la première composition.
P₂ : XIRONA BLUE de la société MERCK pour la deuxième composition.
P₃ : XIRONA GREEN de la société MERCK pour la troisième composition.
P₄ : XIRONA SILVER de la société MERCK pour la quatrième composition de la gamme.
** METASHINE ME 2040 PS de la société NIPPON SHEET GLASS

Pour chaque composition de la gamme, on peut l'associer à une composition de recouvrement ou de base. On décrit ci-dessous des exemples d'associations.

Exemple 8 : Rouge à lèvres

Première composition

| | |
| --- | --- |
| Tri-mellitate de tri-décyle | 11 |
| Lanoline liquide | 10 |
| Malate de d'iso-stéaryle | 13 |
| Lanoline acétylène | 10 |
| Triglycérides d'acides laurique/palmitique/cetylique/stéarique (50/20/10/10) | 5 |
| Cire microcristalline (C20-C60) | 3 |
| Lanolate d'isopropyle protégé | 10 |
| Octyl-2-decanol | 16 |
| Phényl triméthylsiloxy trisiloxane (VISCOSITE: 20 CST - PM: 372) | 4 |
| Cire de polyéthylène (PM :500) | 8 |
| Pigment interférentiel multicouche* | 10 |
| * TIMIRON SILK RED de la société MERCK | |

Deuxième composition (de recouvrement)

| | |
| --- | --- |
| Octyl-2 dodécanol | 0 |
| Ditertiobutyl 4-hydroxytoluène | ,07 |
| Polybutène (monooléfines / isoparaffines 95 / 5) (PM : 2060) | 0 |
| Mélange de p-hydroxybenzoates d'isopropyle, iso-butyle, n-butyle (40/30/30) | ,4 |
| Tétra-iso-stéarate de pentaérythrityle | 1,33 |
| Tri-mellitate de tridécyle | 2 |
| Triglycéride d'acide 2-décyl tétradécanoïque (GUERBET C24) | 1 |
| Pigment à effet** | |
| ** METASHINE ME 2040 PS de la société NIPPON SHEET GLASS | |

La première composition permet de générer une couche de maquillage homogène d'une couleur rouge très intense. La seconde composition permet de créer un effet loupe agrémenté de point de surbrillance argent qui donnent du relief à l'ensemble du résultat maquillage.

Exemple 9 : Rouge à lèvres

Composition de base

| | |
|---|---|
| Tri-mellitate de tri-décyle | 12 |
| Lanoline liquide | 11 |
| Malate de d'iso-stéaryle | 14 |
| Lanoline acétylène | 11 |
| Triglycérides d'acides laurique/palmitique/cétylique/stéarique (50/20/10/10) | 6 |
| Cire microcristalline (C20-C60) | 3 |
| Lanolate d'isopropyle protégé | 10 |
| Octyl-2-decanol | 16 |
| Phényl triméthylsiloxy trisiloxane (VISCOSITE: 20 CST - PM: 372) | 4 |
| Cire de polyéthylène (PM :500) | 8 |
| Pigment noir d'oxyde de Fer $Fe_3O_4$ | 5 |

Composition contenant le pigment interférentiel multicouche

| | |
|---|---|
| Octyl-2 dodécanol | 10 |
| Ditertiobutyl 4-hydroxytoluène | 0,07 |
| Polybutène (monooléfines / isoparaffines 95 / 5) (PM : 2060) | 45,2 |
| Mélange de p-hydroxybenzoates d'isopropyle, iso-butyle, n-butyle (40/30/30) | 0,4 |
| Tétra-iso-stéarate de pentaérythrityle | 11,33 |
| Tri-mellitate de tridécyle | 12 |
| Triglycéride d'acide 2-décyl tétradécanoïque (GUERBET C24) | 11 |
| Pigment interférentiel multicouche** | 10 |
| ** TIMIRON SILK RED de la société MERCK | |

La composition de base est appliquée en premier et permet de générer une couche de maquillage homogène d'une couleur noire très intense. La composition contenant le pigment interférentiel multicouche est appliquée ensuite et permet de créer un effet coloré donnant un résultat maquillage très intense.

Bien entendu, l'invention n'est pas limitée aux exemples qui viennent d'être donnés. L'expression « comportant un » est synonyme de « comportant au moins un » et « compris entre » s'entend bornes incluses.

**Revendications**

1. Gamme de compositions cosmétiques comportant au moins deux compositions comportant chacune un milieu cosmétiquement acceptable et au moins un pigment interférentiel multicouche, chaque composition étant de couvrance supérieure ou égale à 25,
les pigments interférentiels multicouche respectifs des différentes compositions étant choisis de manière à ce que les couleurs des compositions dans la masse, prises deux à deux, diffèrent d'un écart $\Delta E_{\text{avant application}}$ inférieur ou égal à 3 et que les compositions après application présentent, prises deux à deux, un écart de couleur $\Delta E_{\text{après application}}$ supérieur ou égal à 5.

2. Gamme selon la revendication 1, avec $\Delta E_{\text{avant application}} \leq 2$.

3. Gamme selon la revendication 1 ou 2, les compositions de la gamme comportant chacune au moins un pigment interférentiel multicouche ayant un substrat de verre, silice, mica, alumine ou métal.

**4.** Gamme selon l'une quelconque des revendications 1 et 3, les compositions de la gamme comportant chacune au moins un pigment interférentiel multicouche ayant un substrat comportant un oxyde de fer ou d'aluminium.

**5.** Gamme selon l'une quelconque des revendications 1 à 4, les pigments interférentiels multicouche des compositions de la gamme ayant un substrat de même nature.

**6.** Gamme selon l'une quelconque des revendications 1 à 5, toutes les compositions de la gamme ayant les mêmes composés hormis les pigments interférentiels multicouche respectifs.

**7.** Gamme selon l'une quelconque des revendications 1 à 6, toutes les compositions de la gamme étant sensiblement blanches dans la masse.

**8.** Gamme selon l'une quelconque des revendications 1 à 7, les compositions de la gamme ne comportant pas d'autres agents de coloration que les pigments interférentiels multicouche.

**9.** Gamme selon l'une quelconque des revendications 1 à 8, les compositions comportant chacune moins de 0,5% en masse de pigments générant une couleur par absorption.

**10.** Gamme selon la revendication 9, les compositions de la gamme contenant chacune moins de 0,5% en masse de pigments à base d'oxyde de fer.

**11.** Gamme selon l'une quelconque des revendications précédentes, le pigment interférentiel multicouche étant dépourvu de couche colorée.

**12.** Gamme selon l'une quelconque des revendications précédentes, le ou les pigment(s) interférentiel(s) multicouche ne comportant, sur le substrat, qu'une ou plusieurs couches choisies parmi $TiO_2$, $ZrO_2$, $SnO_2$, $SiO_2$.

**13.** Gamme selon l'une quelconque des revendications précédentes, le ou les pigment(s) interférentiel(s) multicouche étant dépourvu(s) de $Fe_2O_3$.

**14.** Gamme selon l'une quelconque des revendications 1 à 13, la teneur massique en pigment interférentiel multicouche dans chaque composition étant supérieure à 7%.

**15.** Gamme selon l'une quelconque des revendications 1 à 14, deux compositions au moins de la gamme comportant des pigments interférentiels multicouche ne différant entre eux que par l'épaisseur d'au moins l'une des couches interférentielles.

**16.** Gamme selon l'une quelconque des revendications précédentes, deux compositions au moins de la gamme comportant des pigments interférentiels multicouche ayant des couches de surface de même nature.

**17.** Gamme selon l'une quelconque des revendications précédentes, comportant trois compositions au moins ayant des pigments interférentiels multicouche, les teneurs massiques totales en pigment(s) interférentiel(s) multicouche des trois compositions de la gamme étant respectivement inférieure ou égale à 8, strictement supérieure à 14 et comprise entre 12 et 14.

**18.** Gamme selon la revendication 17, trois compositions comportant le même pigment interférentiel multicouche.

**19.** Gamme selon l'une quelconque des revendications 1 à 16, comportant au moins deux, voire trois ou quatre, compositions ayant des pigments interférentiels multicouche respectifs différents à une même teneur massique.

**20.** Gamme selon l'une quelconque des revendications 1 à 19, toutes les compositions de la gamme étant conditionnées dans des récipients de formes identiques.

**21.** Gamme selon l'une quelconque des revendications précédentes, au moins un pigment interférentiel multicouche d'au moins une composition de la gamme comportant un substrat de silice, mica ou verre.

**22.** Gamme selon l'une quelconque des revendications 1 à 21, les compositions de la gamme étant anhydres.

**23.** Gamme selon l'une quelconque des revendications 1 à 21, les compositions de la gamme étant aqueuses.

**24.** Gamme selon l'une quelconque des revendications 1 à 23, les compositions de la gamme étant non pulvérulentes et la teneur massique totale dans chaque composition en pigment interférentiel multicouche allant de 7 à 20 %, mieux 8 à 15 %.

**25.** Gamme selon l'une quelconque des revendications 1 à 23, les compositions de la gamme étant pulvérulentes et la teneur massique totale en pigment interférentiel multicouche dans chaque composition allant de 40 à 95 %, mieux 40 à 80 %.

**26.** Gamme selon l'une quelconque des revendications 1 à 25, la couleur des compositions de la gamme dans la masse étant blanche.

**27.** Gamme selon la revendication 26, les compositions dans la masse ayant un indice de blancheur supérieur ou égal à 40.

**28.** Gamme selon l'une quelconque des revendications 1 à 27, au moins une composition de la gamme ne comportant pas d'autre agent de coloration que le ou les pigments interférentiels multicouche.

**29.** Gamme selon l'une quelconque des revendications 1 à 28, au moins un pigment interférentiel multicouche d'une composition de la gamme comportant au moins quatre couches.

**30.** Gamme selon l'une quelconque des revendications 1 à 29, au moins un pigment interférentiel multicouche d'au moins une composition de la gamme comportant un substrat en une matière transparente.

**31.** Gamme selon l'une quelconque des revendications précédentes, la couleur produite par au moins un pigment interférentiel multicouche d'au moins une composition de la gamme n'étant pas de longueur d'onde dominante comprise entre 580 et 650 nm.

**32.** Gamme selon l'une quelconque des revendications 1 à 31, chaque composition de la gamme ne comportant qu'un seul pigment interférentiel multicouche.

**33.** Gamme selon l'une quelconque des revendications 1 à 32, au moins une composition de la gamme comportant au moins deux pigments interférentiels multicouche.

**34.** Gamme selon la revendication 33, les deux pigments interférentiels multicouche ayant des couches constituées des mêmes matériaux mais au moins une couche d'un pigment ayant une épaisseur différente d'une couche correspondante de l'autre pigment.

**35.** Gamme de compositions cosmétiques non pulvérulentes, notamment liquides ou en stick, comportant chacune dans un milieu cosmétiquement acceptable un ou plusieurs pigments interférentiels multicouche,
avec pour au moins une composition une teneur massique totale en pigment(s) interférentiel(s) multicouche inférieure ou égale à 9 % et pour une composition une teneur supérieure ou égale à 11 %.

**36.** Gamme selon l'une quelconque des revendications précédentes, chaque composition étant conditionnée avec des moyens indiquant la couleur de la composition après l'application sur des matières kératiniques.

**37.** Gamme de compositions cosmétiques comportant au moins deux compositions comportant chacune un milieu cosmétiquement acceptable et au moins un pigment interférentiel multicouche en une teneur massique supérieure ou égale à 7 %, les pigments interférentiels générant des couleurs différentes à l'application, les pigments interférentiels étant de préférence à substrat non opaque, notamment de verre, silice, mica, et comportant de préférence aucune couche colorée, la teneur en pigment diffusant générant une couleur par absorption dans chaque composition étant de préférence inférieure à 0,5 %.

**38.** Procédé de maquillage des matières kératiniques, comportant l'application sur les matières kératiniques d'une composition d'une gamme selon l'une quelconque des revendications 1 à 37.

**Office européen
des brevets**

**RAPPORT DE RECHERCHE EUROPEENNE**

Numéro de la demande

EP 07 12 0902

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| Y | DE 10 2004 045352 A1 (MERCK PATENT GMBH [DE]) 21 avril 2005 (2005-04-21) * page 2, alinéa 6 - alinéa 9 * * page 4, alinéa 21 - alinéa 22 * * exemples A,B * ----- | 1-38 | INV. A61K8/02 A61K8/19 A61K8/25 A61K8/29 A61Q1/02 A61Q1/04 A61Q1/06 A61Q1/10 A61Q3/02 |
| Y | EP 1 306 412 A (MERCK PATENT GMBH [DE]) 2 mai 2003 (2003-05-02) * page 2, alinéa 9 - alinéa 10 * * exemples A-E * ----- | 1-38 | |
| Y | EP 1 656 927 A (OREAL [FR]) 17 mai 2006 (2006-05-17) * page 2, alinéa 9 - alinéa 11 * * page 4, alinéa 30 - alinéa 38 * * exemples * ----- | 1-38 | |
| Y | DE 102 51 378 A1 (MERCK PATENT GMBH [DE]) 13 mai 2004 (2004-05-13) * page 2, alinéa 8 - alinéa 10 * * exemples * ----- | 1-38 | DOMAINES TECHNIQUES RECHERCHES (IPC) |
| Y | WO 01/51015 A (COLOR ACCESS INC [US]) 19 juillet 2001 (2001-07-19) * le document en entier * ----- | 1-38 | A61K A61Q |
| Y | US 4 743 443 A (PISANI ARTHUR [US] ET AL) 10 mai 1988 (1988-05-10) * le document en entier * ----- | 1-38 | |
| Y | US 4 714 085 A (VON KLEINSORGEN REINHARD [DE]) 22 décembre 1987 (1987-12-22) * le document en entier * ----- | 1-38 | |

Le présent rapport a été établi pour toutes les revendications

| Lieu de la recherche | Date d'achèvement de la recherche | Examinateur |
|---|---|---|
| Munich | 11 avril 2008 | Ruckebusch, Virginie |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 07 12 0902

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

11-04-2008

| Document brevet cité au rapport de recherche | Date de publication | Membre(s) de la famille de brevet(s) | Date de publication |
|---|---|---|---|
| DE 102004045352 A1 | 21-04-2005 | AUCUN | |
| EP 1306412 A | 02-05-2003 | DE 10151844 A1<br>JP 2003183538 A<br>US 2003097965 A1 | 08-05-2003<br>03-07-2003<br>29-05-2003 |
| EP 1656927 A | 17-05-2006 | FR 2877839 A1<br>JP 2006137760 A | 19-05-2006<br>01-06-2006 |
| DE 10251378 A1 | 13-05-2004 | AUCUN | |
| WO 0151015 A | 19-07-2001 | AT 293435 T<br>AU 777557 B2<br>AU 2639301 A<br>CA 2366626 A1<br>DE 60110172 D1<br>DE 60110172 T2<br>EP 1189586 A2<br>ES 2241775 T3<br>JP 2003519644 T<br>US 6428773 B1 | 15-05-2005<br>21-10-2004<br>24-07-2001<br>19-07-2001<br>25-05-2005<br>06-04-2006<br>27-03-2002<br>01-11-2005<br>24-06-2003<br>06-08-2002 |
| US 4743443 A | 10-05-1988 | AUCUN | |
| US 4714085 A | 22-12-1987 | DE 8313487 U1 | 10-11-1983 |

EPO FORM P0460

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 1172091 A

**Littérature non-brevet citée dans la description**

- International Cosmetic Ingredient Dictionnary and Handbook. The Cosmetic, Toiletry, and Fragrance Association, 1997, 371-386524-528